# EUROPEAN PATENT APPLICATION

(11) **EP 4 656 216 A1**
(43) Date of publication of application: **03.12.2025**
(21) Application number: 24382568.4
(22) Date of filing: 28.05.2024
(51) Int. Cl.: A61M 5/168

(54) **MEDICAL THREE-WAY STOPCOCK, MEDICAL FLUID MEASURING DEVICE AND FLUID FLOW MONITORING SYSTEM**

(71) Applicant: Fundación de la Comunidad Valenciana Hospital General para la Investigación Biomédica, Docencia y Desarrollo de las Ciencias de la Salud, 46014 Valencia (ES); AIMPLAS - Asociación de Investigación de Materiales Plásticos y Conexas, 46980 Paterna (ES)
(72) Inventor: ROVIRA SORIANO, Lucas, 46183 LA ELIANA (ES); KOT BAIXAULI, Pablo, 46017 VALENCIA (ES); POZO ORTÍ, Silvia, 46183 LA ELIANA (ES); CANO MARÍN, Beatriz, 46017 VALENCIA (ES); RUEDAS ABARCA, Vicente, 46025 VALENCIA (ES)
(74) Representative: ZBM Patents - Zea, Barlocci & Markvardsen

(57) **Abstract**

A medical fluid measuring device for measuring a fluid supplied to a patient comprising: a conduit comprising an inner diameter of less than 3 mm, wherein the conduit comprises: an inlet for receiving the fluid, and an outlet configured to be connected to a proximal end of a catheter to supply an outlet fluid to a patient; and a flow meter assembly for measuring the outlet fluid flow, wherein the flow meter assembly is configured to measure a fluid flow of less than 100 mL/min.

## Description

The present disclosure relates to a medical three-way stopcock, a medical fluid measuring device and a fluid flow monitoring system for monitoring a patient body fluid balance.

### BACKGROUND

Some medical treatments require the supply of a flow of fluid, such as crystalloids, colloids, blood, serum, nutrients, and medication, to a patient. The fluid may be supplied from a fluid source to the patient through a medical tube such as a conduit, a line, an stopcock, a catheter or any other tube.

Sometimes, a patient may require the supply of different types of fluid at the same time. This supply is possible through the same intravenous catheter or through different catheters. The patient may require the supply of fluid from different fluid sources. Three-way stopcocks allow simultaneous delivery of fluid from two different two fluids sources through a single intravenous catheter. These devices comprise a first inlet for receiving a first fluid, a second inlet for receiving a second fluid, and an outlet to supply an outlet fluid to a patient. An intravenous catheter is usually connected to the outlet to conduct the outlet fluid into the patient.

Impulsion systems can be used as fluid sources to supply fluid to a patient. Impulsion systems such as electronic pumps, manual boluses and other models of pumps can comprise a regulation element to regulate the amount of fluid, and the fluid flow, delivered by them. These impulsion systems can also comprise a flow meter that measures the amount of fluid, and the fluid flow, delivered. This flow meter is generally an integral part of the impulsion system. However, as the fluid flow is measured at the fluid source and not at the entrance to the patient, the amount of fluid supplied to the patient may not exactly correspond to the amount of fluid delivered by the impulsion system. This may lead to an imprecision in the measurement of the fluid supplied to the patient. In addition, during the intravenous treatment of a patient, fluids can also be supplied from fluid sources that do not comprise regulation elements and/or flow meters and thus may not being measured.

Supplying fluid into the patient can alter the body fluid balance of the patient. This balance corresponds to the difference between the fluid inputted and the fluid outputted from the body of the patient. An improper balance of inputs and outputs may end up in hypervolemia, congestion and oedema, if the inputs are much higher than the outputs, or hypervolemia and dehydration, if the outputs are much higher than the inputs, among other complications. Other problems such as hydroelectrolitic alterations also include a risk of high morbidity and mortality. To avoid these complications, the amount of fluid inputted to and outputted from the patient may be measured.

To ensure a proper patient body fluid balance and thus to avoid complications that can risk the patient, medical staff members have to constantly check, quantify, account, and annotate the intravenous fluid intake of the patient and the outputted body fluid. This annotation is usually performed manually, e.g. on a sheet of paper. The task of monitoring the fluid inputted and outputted from the patient is tedious, inefficient and sometimes inaccurate. An inaccurate register of the fluid inputted and outputted from the patient may lead to an improper patient body fluid balance.

In addition, a patient can undergo a critical complication that may require a sudden input of a medical fluid, and this quantity of fluid introduced cannot be precisely quantified due to the celerity of the insertion or by prolonged administration over time. Therefore, in some cases, the staff member can only consider in their annotation a rough estimate of the fluid inputted.

Furthermore, the amount of fluid inputted into the body and outputted from the body is not generally updated and are only measured from time to time. This may complicate the proper evaluation of the balance and therefore the risk of complications on a patient.

The present disclosure provides examples of devices that at least partially resolve some of the aforementioned disadvantages.

### SUMMARY

In a first aspect, a medical three-way stopcock is provided. The medical three-way stopcock comprises a main body. The main body comprises a first inlet for receiving a first fluid and a second inlet for receiving a second fluid. The main body comprises an outlet. The main body comprises a central part fluidically connecting the first inlet and the second inlet to the outlet for blending the first fluid and the second fluid to obtain an outlet fluid. The outlet is configured to supply the outlet fluid to a patient. The medical three-way stopcock comprises a flow meter assembly for measuring an outlet fluid flow.

The first inlet, the second inlet and the outlet can receive fluids from internal sources of the patient towards the outside of the patient (outflow direction) in addition to receiving fluids form external sources towards the inside of the patient (inflow direction). The outlet fluid flow measured by the flow meter assembly can correspond to the fluid supplied to the stopcock by at least one of the first inlet or the second inlet and that exits the stopcock by the outlet of the stopcock. In addition, the outlet fluid flow measured by the flow meter assembly may correspond to fluid emanated from the patient to the stopcock that circulates through the stopcock and exits the stopcock though at least one of the first inlet or the second inlet.

Therefore, the three-way stopcock of the disclosure allows measuring the amount of outlet fluid delivered into the patient and also allows measuring the amount of outlet fluid emanating from the patient, e.g. blood. The amount of outlet fluid, e.g fluid delivered to the patient or emanated from the patient, may thus be precisely measured, even when fluid is supplied from different fluid sources connected to the inlets of the three-way stopcock, or when fluid is emanated from the patient and exists the stopcock by both the first inlet and the second inlet.

As the outlet is the part of the stopcock more proximal to the patient, the measurement of the outlet fluid flow supplied by the outlet is more accurate than measuring the fluid at inlets or before reaching the inlets. Measuring the flow at the outlet is more accurate than a measurement performed by flow meters placed at impulsion systems that supply fluid into the stopcocks, as some fluid flow losses may undesirably occur while conducting the fluid from them to the stopcock. In addition, measuring the outlet fluid flow supplied by the outlet is simpler than measuring the amount of stopcock input fluid inputted to the first inlet and to the second inlet, as both measurements would have to be totalled to obtain the amount of outlet fluid. This may be even more tedious when fluid vials are connected to the inlets as these fluid vials must be replaced frequently.

In an example, the outlet comprises an inner diameter of less than 3 mm. In an example, the first inlet and/or the second inlet comprise an inner diameter of less than 3 mm. In an example, the flow meter assembly is configured to measure a fluid flow of less than 100 mL/min. The flow meter assembly is configured to measure fluid flows that have low fluid flow and that are at low pressure. The flow meter assembly can therefore measure the fluid flow corresponding to fluid flows commonly employed during medical treatments. Medical treatments in which a flow of fluid is supplied commonly have a fluid flow of about 0.01-0.1 mL/min (0.6-60 mL/hour), which maximum fluid flows in the range of between 24-100 mL/min.

In a second aspect, a medical fluid measuring device for measuring a fluid supplied to a patient is provided. The medical fluid measuring device comprises a conduit comprising an inner diameter of less than 3 mm. The conduit comprises an inlet for receiving the fluid, and an outlet configured to be connected to a proximal end of a catheter to supply an outlet fluid to a patient. The medical fluid measuring device also comprises a flow meter assembly for measuring the outlet fluid flow. The outlet fluid is the fluid supplied to the patient. The flow meter assembly is also configured to measure a fluid flow of less than 100 mL/min. The flow meter assembly can therefore measure the fluid flow corresponding to fluid flows commonly employed during medical treatments.

The medical fluid measuring device of the second aspect of the disclosure allows measuring of the amount of outlet fluid delivered into the patient. This device can be connected to any medical tube, for example, a line, a conduit or a catheter corresponding to any fluid source.

The inlet of the conduit is configured to receive the fluid via a medical tube regardless of its characteristics, for example the medical tube being a medical tube from an electronic pump, a manual bolus, a corporal fluid, etc. The outlet is configured to be connected to a standard catheter such as an intravenous catheter. Therefore, the medical fluid measuring device of the second aspect is configured to be connected to a medical tube that delivers fluid and to a catheter to supply an outlet fluid to a patient. The medical fluid measuring device element acts as a connector element configured to connect a medical tube with a catheter. Therefore, the medical fluid measuring device can be referred as a connector or as an intermedial conduit. The amount of outlet fluid delivered to the patient, i.e. fluids entering the patient's body may thus be precisely measured regardless of the fluid source from which the fluid is supplied to the measuring system and thus received by the inlet. Thus, the amount of fluid delivered from a fluid source such as impulsion system can be measured more proximal to the patient, therefore obtaining a more accurate measurement. In addition, the device of the second aspect can be connected to a single fluid source. In an example, the inlet of the second aspect comprises an inlet connector for the connection with a medical tube. In an example, the outlet comprises an outlet connector for the connection with a catheter.

The inlets of both the medical three-way stopcock of the first aspect and the medical fluid measuring device of the second aspect can receive fluid regardless of the impulsion system used (electronic pump, manual boluses, gravity pump system), and regardless of the type of administration of fluid (e.g. sequential or parallel administration).

The medical fluid measuring device of the second aspect, as well as the medical three-way stopcock of the first aspect, can be easily connected to any tube, catheter, or conduit for the injection and supply of fluid into a patient, such as an intravenous catheter. By virtue of the connection, the three-way stopcock of the disclosure, as well as the medical fluid measuring device of the second aspect, can be used with any kind of fluid source and can measure fluids that have not been impulsed by them. This is an advantage over flow meters that are integrated on corresponding impulsion systems, as these flow meters can only measure the volume that is administered from their own impulsion system, but do not quantify other fluids that have not been impulsed by them. Also, in the occasions when a sudden input of a medical fluid is needed, and no time can be spent in preparing a pump with a regulation element or with a flow meter to pump the fluid into the patient, the utilization of the medical three-way stopcock of the first aspect and the medical fluid measuring device of the second aspect provide an accurate measurement of the fluid inputted into a patient.

The control of the outlet fluid improves the control of the patient body balance by medical staff member, improving their evaluation of whether the total body fluid of the patient is below a threshold value or above a threshold value. Based on their evaluation, the staff member can modify the flow of one or more inlet fluids to avoid possible complications on the patient.

In addition, the manufacture of both the stopcock and measuring device of the disclosure is cheaper than the regulation elements of impulsion systems used as fluid sources to supply and deliver fluid. Therefore, the medical three-way stopcock of the disclosure allows an accurate quantifying of the amount of outlet fluid supplied to a patient without an elevated economic cost.

In some examples of both the first aspect and/or the second aspect, the flow meter assembly comprises an outer housing removably attached to the main body of the medical three-way stopcock or to the conduit of the medical fluid measuring device. Stopcocks and conduits used in medical treatments are usually discarded after each use. The outer housing allows both the stopcock of the first aspect and the device of the second aspect to have a detachable part. Therefore, a part of the stopcock corresponding to the main body of the stopcock can be discarded after each use, while another part of the stopcock can be reused. Analogously, a part of the device corresponding to the conduit can be discarded after each use, while another part of the device can be reused. In some examples, the flow meter assembly comprises a detectable element and a sensor to detect the presence of the detectable element. In some examples, the stopcock of the first aspect and the device of the second aspect comprise a flow meter controller. Advantageously, the more expensive elements of the stopcock, such as the detectable element, the sensor, and/or the flow meter controller, can be placed in the outer housing so they can be detached from the main body after each use and therefore be reused. The flow meter controller may be configured to receive data from the sensor, and determine, based on the data received from the sensor, an amount of output fluid supplied by the outlet during a period of time.

In some examples, the flow meter assembly of the first aspect and/or the second aspect further comprises a user interface placed in the outer housing. The user interface enables the indication of the amount of stopcock output fluid that circulates through the outlet, which helps the medical staff member to properly evaluate the body fluid balance of the patient.

In some examples, the flow meter assembly of the first aspect and/or second aspect comprises a movable element. The movable element may be configured to be contacted by the outlet fluid, i.e. the fluid outputted from the three-way stopcock and/or the medical fluid measuring device. The movable element may be rotated about a movable element rotating axis. The outlet fluid may thus cause the rotation of the movable element about the movable element rotating axis.

In some of these examples, a detectable element may be placed in the movable element. The sensor may detect the detectable element mounted on the movable element. The number of rotations of the movable element may thus be detected by the sensor.

In other examples, the movable element may drive an input gear of a gear assembly.

The rotation of the movable element caused by the outlet fluid may thus drive the rotation of the input gear. The gear assembly may be configured to reduce the number of rotations from the input gear to an output gear. The output gear of the gear assembly may drive an outlet fluid visual indicator. In these examples, a sensor to detect the presence of a detectable element, and consequently, to measure the outlet fluid, is not required. Accordingly, the flow meter assembly of these examples may be more cost-effective.

In a third aspect of the present disclosure, a fluid flow monitoring system for monitoring a patient body fluid balance is provided. The fluid flow monitoring system comprises one or more medical three-way stopcocks according to any of the examples of the first aspect and/or one or more medical fluid measuring devices according to any examples of the second aspect. The fluid monitoring system further comprises one or more body patient output fluid meters for measuring the flow of fluid outputted from the patient body. In addition, the fluid monitoring system comprises a monitoring system controller.

An advantage of the fluid flow monitoring system is that the fluid flow monitoring system can collect and integrate several medical three-way stopcocks and/or medical fluid measuring devices. Each measurement device can measure the flow of fluid of each catheter inserted into the patient. Therefore, the fluid flow monitoring system can add inputs and outputs to calculate the patient body balance. The fluid flow monitoring system allows monitoring all the fluid that passes through the system. Therefore, it monitors all the fluid that passes through the one or more three-way stopcocks and/or the one or more medical fluid measuring devices.

By virtue of comprising one or more medical three-way stopcocks according to any of the examples of the first aspect and/or one or more medical fluid measuring devices according to any examples of the second aspect, the medical fluid monitoring system has the advantage of allowing the measurement of any fluid regardless of the impulsion system used (electronic pump, manual boluses, gravity pump system, etc.) and regardless of the type of administration (e.g. sequential or parallel administration). Another advantage is that as the inlets of both the three-way stopcocks and the medical fluid measuring devices can receive fluids both from external sources towards the inside of the patient (inflow) and from internal sources of the patient towards the outside of the patient (outflow), one or more medical three-way stopcocks according to any of the examples of the first aspect and/or one or more medical fluid measuring devices according to any examples of the second aspect can be used as a body patient output fluid meters for measuring the flow of fluid outputted from the patient body.

The monitoring system controller is configured to obtain an amount of output fluid supplied by the outlet of the one or more medical fluid measuring devices and/or by the one or more medical three-way stopcocks during a period of time; determine, based on the obtained amount of outlet fluid, an amount of fluid inputted into the patient body during the period of time; obtain, from the one or more body patient output fluid meters, an amount of fluid outputted from the patient body during the period of time; compare the amount of fluid inputted into the patient body during the period of time with the amount of fluid outputted from a patient body during the period of time; and determine, based on the comparison, the patient body fluid balance during the period of time.

To ensure the body fluid balance and to avoid complications that can risk the patient, the fluid inputted and outputted from the patient is controlled and monitored. Contrary to the typical monitorization performed manually by a medical staff member, the body fluid balance monitoring system according to the third aspect of the present disclosure allows a more reliable monitoring of the body fluid balance.

The fluid flow monitoring system allows a simpler, precise, and more accurate monitorization of the body patient fluid balance. The balance also allows an individualized balance in real time for each patient. This monitorization allows the medical staff member to control the patient body fluid balance and to determine the adequacy of the patient current treatment. The control and monitoring can be performed in real time. Therefore, the medical staff member can access updated information of the balance at any moment.

In some examples, the monitoring system controller is further configured to compare the patient body fluid balance with a predetermined patient body fluid balance upper threshold value and with a predetermined patient body fluid balance lower threshold value. This comparison allows an easier determination of whether the patient body fluid balance at a certain moment may imply a risk for a patient. Therefore, this comparison allows the medical staff member to modify the flow of fluid delivered into the stopcocks, and therefore to the patient. The risk of having complications to the patient may thus be reduced. Additionally, or alternatively, the medical staff member may decide, for example, the input or output of additional fluid into the patient, e.g. by feeding the patient or by providing the patient with diuretic medication.

### BRIEF DESCRIPTION OF THE DRAWINGS

Non-limiting examples of the present disclosure will be described in the following, with reference to the appended drawings, in which:
Figure 1A schematically illustrates a view of a medical stopcock according to an example of the first aspect of the present disclosure.
Figure 1B is a cross-sectional view of the medical stopcock of Figure 1A.
Figure 1C schematically illustrates a view of a medical stopcock according to an example of the first aspect of the present disclosure, in which medical tubes are connected to the stopcock.
Figure 2A schematically illustrates a view of a medical stopcock according to an example of the first aspect of the present disclosure, in which the flow meter assembly comprises an outer housing.
Figure 2B schematically illustrates a cross-sectional view of the medical stopcock of Figure 2A.
Figure 3 schematically illustrates a cross-sectional view of a medical stopcock according to an example of the first aspect of the present disclosure, in which the detectable element is placed inside the outlet.
Figure 4 schematically illustrates a cross-sectional view of a medical stopcock according to an example of the first aspect of the present disclosure, in which the detectable element is placed outside the outlet.
Figure 5A schematically illustrates a cross-sectional view of a medical stopcock according to an example of the first aspect of the present disclosure, in which the stopcock comprises an outlet fluid visual indicator and a gear assembly.
Figure 5B schematically illustrates a cross-sectional view of a medical stopcock according to an example of the first aspect of the present disclosure, in which the stopcock comprises an outlet fluid visual indicator and a gear assembly.
Figure 6 schematically illustrates a view of a medical fluid measuring device according to an example of the second aspect of the present disclosure.
Figure 7 schematically illustrates a view of a medical fluid measuring device according to an example of the second aspect of the present disclosure, in which the flow meter assembly comprises an outer housing.
Figure 8 schematically illustrates a cross-sectional view of a medical fluid measuring device according to an example of the second aspect of the present disclosure, in which the detectable element is placed inside the outlet.
Figure 9 schematically illustrates a cross-sectional view of a medical fluid measuring device according to an example of the second aspect of the present disclosure, in which the detectable element is placed outside the outlet.
Figure 10 schematically illustrates a flow monitoring system according to an example of the third aspect of the present disclosure.

### DETAILED DESCRIPTION OF EXAMPLES

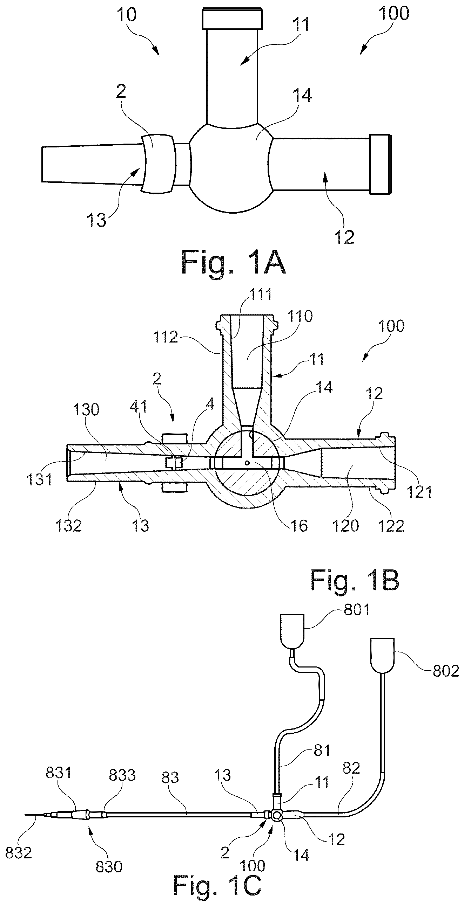

Figures 1A and 1B illustrate an example of a medical stopcock 100 according to the present disclosure. The medical three-way stopcock 100 comprises a main body 10. The main body 10 comprises a first inlet 11 and a second inlet 12 for respectively receiving a first fluid and a second fluid. The main body 10 further comprises an outlet 13 configured to supply an outlet fluid to a patient. The main body 10 also comprises a central part 14 fluidically connecting the first inlet 11 and the second inlet 12 to the outlet 13 for blending the first fluid and the second fluid to obtain an outlet fluid. The outlet fluid is thus the fluid outputted from the outlet 13 towards the patient's body. The inlets 11, 12 can also be referred to as upstream openings of the three-way stopcock, while the outlet 13 can also be referred to as a downstream opening of the three-way stopcock 100. The outlet fluid is the sum, or the total, of the first fluid inputted to the stopcock 100 through the first inlet 11 and the second fluid inputted to the stopcock 100 through the second inlet 12 after both first fluid and second fluid are blended.

The first inlet 11 is configured to be connected to a first inlet line, tube, or conduit. Fluid from a fluid source, such as a pump or a fluid vial, can be inputted to the first inlet 11. Similarly, the second inlet 12 is configured to be connected to a second inlet line to input a second fluid from a fluid source to the second inlet 12. When a medical tube, e.g. a line, a tube, a catheter, a fluid vial or a conduit, is connected to the first inlet 11 and/or the second inlet 12, a flow of fluid can be delivered, respectively, from the fluid source into the first inlet 11 and/or the second inlet 12. Fluid entering through first inlet 11, i.e. first fluid, and from the second inlet 12, i.e. second fluid, are blended in the central part to form the outlet fluid to be outputted by the outlet 13. The outlet fluid thus corresponds to the mix of the first fluid with the second fluid.

The outlet 13 is configured to be connected to a line, a tube, a catheter, or a conduit to deliver the outlet fluid to a patient. The first inlet 11, the second inlet 12 and the outlet 13 of this example have a generally cylindrical and tubal shape. This shape eases the connection with a cylindrical tube, e.g. a catheter.

Figure 1B illustrates a cross-section of the stopcock 100 of Figure 1A. The first inlet 11 comprises an external surface 112, an internal surface 111, and a fluid passageway 110 defined by the internal surface 111 of the first inlet. The inlets extend an inlet length from an inlet proximal end to an inlet distal end. Analogously, the second inlet 12 comprises an external surface 122, an internal surface 121 and a fluid passageway 120 defined by the internal surface 121 of the second inlet 12. The outlet 13 of this example also comprises an external surface 132 and an internal surface 131 defining a fluid passageway 130. The outlet 13 extends an outlet length from an outlet proximal end to an outlet distal end.

The main body further comprises a central part 14. The proximal ends of the inlets and of the outlets are connected to the central part 14. The central part 14 comprises a blending area 16, fluidically connected to the first inlet fluid passageway 110, the second inlet fluid passageway 120 and to the outlet fluid passageway 130. The first fluid, which is received by the first inlet 11, and the second fluid, which is received by the second inlet 12, are blended in the blending area 16 to obtain an outlet fluid. The outlet fluid flows through the outlet fluid passageway 130 to be supplied to the patient.

The medical stopcock 100 also comprises a flow meter assembly 2 for measuring the flow of outlet fluid. By measuring the flow of outlet fluid, the stopcock 100 allows the determination of the amount of fluid supplied to a patient. The flow of fluid can be measured in real-time.

The flow meter assembly 2 of this example comprises a movable element 4 configured to be contacted by the outlet fluid for moving the movable element 4. In other examples, an actuator may be pushed by the outlet fluid and the movement of the actuator may drive the movement of the movable element.

In the example of this figure, the contact of the movable element 4 with the outlet fluid causes a movement of the movable element 4 placed inside the outlet 13. The movement of the movable element 4 allows the measurement of the outlet fluid. The expression "placed inside the outlet" is to be understood as placed in the outlet fluid passageway 130. For example, the movable element 4 may be attached to the internal surface 131 of the outlet 13.

The expression "outside the outlet" is to be understood as placed at the external surface 132 of the outlet 13 or in a part of the main body 10 different from the outlet 13, e.g. the central part 14, or one of the inlets 11, 12.

The flow meter assembly 2 of this example is arranged at the outlet 13, The flow meter assembly 2 of this figure is attached to the outlet 13. The placement of the flow meter assembly 2 at the outlet 13 eases the measurement of the flow of outlet fluid. In the example shown in Fig 1B, the movable element 4 is inside the outlet 13. In this example, the movable element 4 is configured to rotate about a movable element rotating axis. The movable element 4 of this example comprises a movable element rotating shaft 41 extending along the movable element rotating axis. In this particular example, the movable element 4 is a plate and has a generally parallelepipedal shape. In other examples, the position of the movable element 4 or the shape of the movable element 4 can be different.

Figure 1C shows the connection of the medical three-way stopcock 100 for the intravenous supply of different types of fluid to a patient.

The first inlet 11 of the stopcock 100 is connected to an end of a first inlet line 81. The first inlet line may be a tube conducting a first fluid. The first inlet line 81 fluidically connects the first inlet 11 with a first fluid source 801, e.g. a pump, to deliver the first fluid into the stopcock 100. Analogously, the second inlet 12 of the stopcock 100 is connected to an end of a second inlet line 82, e.g. a tube. The second inlet line 81 fluidically connects the second inlet 12 with a second fluid source 802.

The outlet 13 of the stopcock 100 is connected to an outlet line 83. The outlet line 83 fluidically connects the outlet 13 with an intravenous catheter 830. The intravenous catheter 830 allows the delivery and supply of fluid into a patient. The fluid supplied may be any type of fluid, such as blood, serum, nutrients, and medication. The intravenous catheter 830 is a catheter of any known type and is not an object of the present disclosure. In the example shown, the intravenous catheter 830 comprises a tip 832 or needle for insertion into the vein, a central part 831 with a handle, and an end 833 for the connection with the outlet line 83.

When a first fluid and a second fluid are delivered from a first fluid source 801 and a second fluid source 802, the fluids are received at the first and second inlets 11, 12 of the stopcock and circulated through corresponding fluid passageways 110, 120 into the central part 14 of the outlet. The first fluid and the second fluid are blended at the blending area 16 of the central part 14 of the stopcock 100, obtaining a stopcock output fluid. The stopcock output fluid circulates through the fluid passageway 130 of the outlet 13. The outlet 13 supplies the outlet fluid to a patient through the outlet line 83 and the catheter 830. The flow meter assembly 2 measures the outlet fluid, i.e. the total fluid supplied by the catheter 830. This measurement allows the quantification of the amount of fluid delivered into the patient during a predetermined period of time. This measurement also allows the quantification of the flow of the outlet fluid delivered to the patient.

The outlet 13 comprises an inner diameter of less than 3 mm. In addition, the inlets 11, 12 also comprise an inner diameter of less than 3 mm. Therefore, the outlet 13 of the stopcock 100 is configured to supply a low fluid flow. For example, the outlet 13 of the stopcock 100 is configured to supply a fluid flow of less than 100 mL/min. Accordingly, the flow meter assembly 2 is configured to measure a fluid flow of less than 100 mL/min.

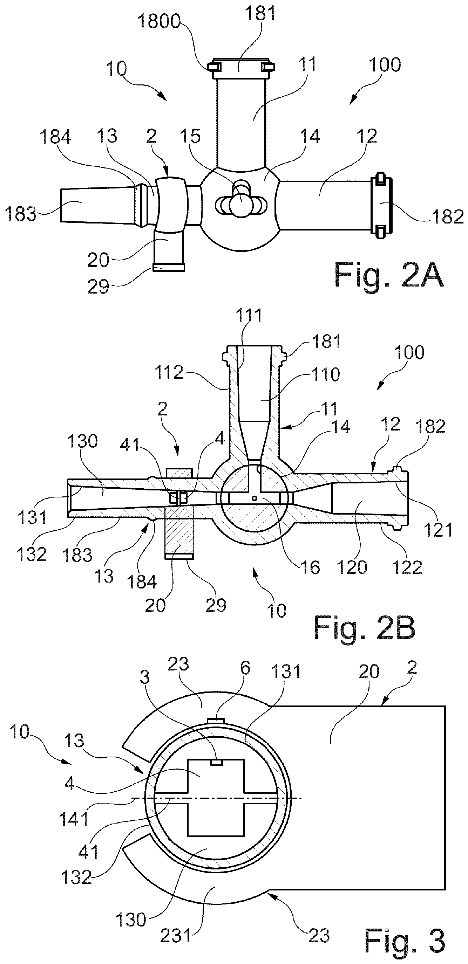

Figures 2A and 2B illustrate a three-way stopcock 100 according to an example of the present disclosure. The flow meter assembly 2 of this example comprises an outer housing 20 according to any example of outer housing of the first and/or the second aspect of the present disclosure.

The outer housing 20 of this example is removably attached to the main body 10, more precisely to the outlet 13. This removable attachment allows a part of the flow meter assembly 2, corresponding to the outer housing 20, to be detachable.

In this figure, the movable element 4 is placed inside the outlet 13. Other components may be placed in the outer housing 20, such as for example a detectable element, a sensor, a controller and/or a user interface. The outer housing 20 is configured to be removably attached to another main body to reuse the outer housing 20, while the main body 10 is a fungible component. The outer housing 20 and the elements arranged at outer housing 20 can therefore be reused after each use of the stopcock 100. Only the components of the flow meter assembly 2 arranged inside the outlet 13, such as, in this example, the movable element 4, are to be discarded with the main body 10 of the stopcock 100 after each use of the stopcock 100.

In the example of Figures 2A and 2B, the main body 10 comprises a valve 15 to enable and disable the pass of the first fluid and the second fluid into the blending area 16 of the central part 14 of the main body 10. The valve 15 is placed in the central part 14 of the main body 10. The valve 15 allows the control of the opening and closing of the flow of fluid received in each of the inlets 11, 12. The valve 15 therefore enables and disables the pass of the first fluid and the second fluid into the central part 14 of the main body 10. The valve 15 also allows closing the system like a plug when it is not in use. In addition, the stopcock may comprise a non-return valve (not shown) placed in the first inlet and/or in the second inlet and/or the outlet to ensure that the fluid flows through the stopcock in a correct direction (from the inlets to the outlet), such as described according to the second aspect of the disclosure.

The flow meter assembly 2 of this example comprises a user interface 29 placed in the outer housing 20. The user interface may be used to control the operation of the stopcock 100. For example, the user interface device may be used to control the valve 15. In addition, the user interface can provide a visualization of the measurement of the outlet fluid made by the flow meter assembly 2.

In this example, the first inlet 11 of the stopcock 100 comprises an inlet connector 181 configured to engage a first inlet line 81. Analogously, the second inlet 12 comprises an inlet connector 182 configured to engage a second inlet line 82. The inlet connector 181, 182 may enhance the connection of the lines 81, 82 with their respective inlets 11, 12. The inlet connectors 181, 182 of this example comprise protrusions 1800 for further fixing the inlet lines 81, 82 into the inlet connectors 181, 182. The outlet 13 also comprises an outlet connector 183 configured to engage an outlet line 83. The outlet connector 183 may be configured for the connection of a catheter. In this example, the outlet connector 183 comprises a protrusion part 184 to cause a dimensional interference with an end of the output line or with the proximal end of a catheter; and the external surface 132 of the outlet 134 has an external diameter such that the inner surface of an output line is fixed outside the external surface 132 of the outlet.

Figure 3 illustrate shows a cross-sectional view of a three-way stopcock comprising a flow meter assembly according to an example of the first and/or the second aspect of the present disclosure.

In the example of Figure 3, the flow meter assembly 2 comprises a detectable element 3 and a sensor 6 to detect the presence of the detectable element 3. The sensor 6 detects the detectable element 3 when the detectable element 3 is placed close enough to the sensor 6. The detectable element 3 of this example is placed in the movable element 4. The detectable element 3 of this example is attached to the movable element 4 linked and/or forming an integral part of the movable element 4. In other examples, the detectable element may form an integral part of the movable element 4. In these examples, the movement of the movable element may thus cause the detectable element 3 to move together with the movement of the movable element 4. In other words, the movement of the movable element 4 moves the detectable element 3. The sensor 6 detects the detectable element 3 when the movement of the movable element 4 leads the detectable element 3 to a position close enough to the sensor 6 to be detected. This detection allows the flow meter assembly 2 to measure the flow of the outlet fluid. The amount of outlet fluid passing through the movable element may thus be detected.

In Figure 3, the detectable element 3 and the movable element 4 are placed inside the outlet 13. The detectable element 3 and the movable element 4 are thus arranged at the outlet passageway 130. The movable element 4 may be contacted by the outlet fluid. The contact of the movable element 4 by the outlet fluid causes a movement of the movable element 4, and consequently of the detectable element 3.

In the example of Figure 3, the movable element 4 is configured to rotate about a movable element rotating axis 141. The movable element 4 comprises a movable element rotating shaft 41 extending along the movable element rotating axis 141. In this configuration, the flow of outlet fluid causes a rotation of the movable element 4 around the movable element rotation axis. The detectable element 3 may thus rotate around the movable element rotation axis 141. The sensor 6 may then detect the position of the detectable element 3. In this example, the movable element rotating axis 141 and the movable element rotating shaft 41 are perpendicular to the outlet length.

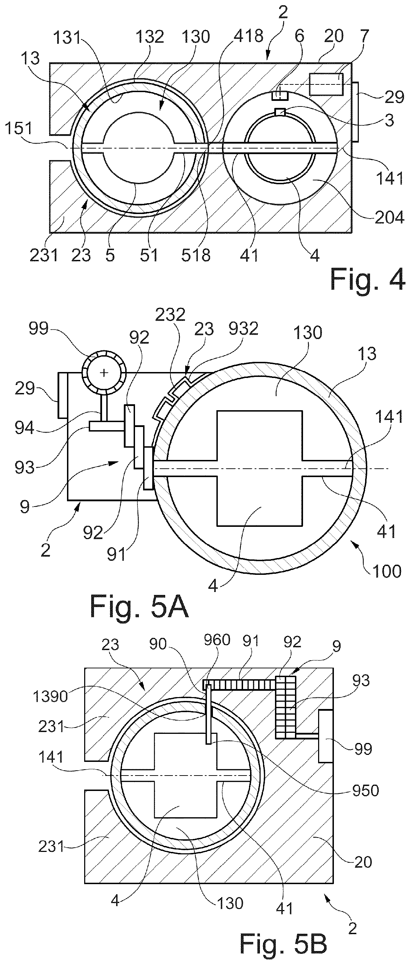

The movable element 4 of Figure 3 is a plate having a parallelepipedal shape. Alternatively, the movable element can be a ring (such as shown in Figure 4), a disc, a rotor, a helix, a turbine or any other shape such as to allow the movable element to rotate about a movable element rotation axis.

In this example, the detectable element 3 is arranged at the perimeter of the movable element 4. The detectable element 3 is an electromagnet, and the sensor 6 is an electromagnetic sensor. The sensor 6 may be a Hall effect sensor. Alternatively, other types of detectable elements 3 and sensors 6 can be used. In an example, the movable element 4 is a turbine comprising a rotor. When the fluid moves the turbine, the rotor rotates, changing the rotation speed. This speed is then detected by a sensor, that outputs a pulse signal that can be send to a controller to evaluate the fluid flow. The examples of flow meter assembly as described in relation to the stopcock of the first aspect may analogously be applied to the medical fluid measuring device according to the second aspect.

The flow meter assembly 2 of Figure 3 comprises an outer housing 20 removably attached to the main body. Due to being removably attached, a part of the flow meter assembly 2 corresponding to the outer housing 20, is detachable from the main body 10 of the stopcock 100. The sensor 6 of this example is in the outer housing 20. This allows the sensor 6 to be reused after using the stopcock 100.

The outer housing 20 comprises an engaging portion 23 to removably engage the main body 10. In the example shown, the engaging portion 23 is configured to removably engage the outlet 13 and is configured to at least partially surround the outlet 13. The main body 10 can also comprise an engaging portion to removably engage the engaging portion of the outer housing. The engaging portion 23 of the outer housing 20 and the engaging portion of the main body 10 forms a snap-fit connection. The engaging portion 23 of Figure 3 comprises a pair of fitting surfaces 231 to press an engaging portion of the main body. The outlet 13 may thus be clamped by the pair of fitting surfaces 231. The engaging portion of the main body of this example is the external surface 132 of the outlet 13. Alternatively, the engaging portion 23 may be a clipping portion, may comprise a protrusion or a recess to engage with a protrusion or a recess on the main body 10 of the stopcock, or may have any other engaging means. The examples of outer housing 20 and engaging portion 23 illustrated in Figure 3 in relation to the stopcock of the first aspect may analogously be applied to the medical fluid measuring device according to the second aspect.

Figure 4 shows a cross-sectional view of a flow meter assembly according to an example of the first and/or the second aspect of the present disclosure. Contrary to Figure 3, in this figure the detectable element 3 mounted on the movable element 4 is arranged outside the outlet 13.

The outer housing 20 of this example is removably attached to the main body and houses the movable element 4, the detectable element 3 and the sensor 6. These elements are thus arranged outside the main body 10. In this example, the outer housing comprises a cavity 204 to accommodate the movable element 4.

As explained regarding Figure 3, the detectable element 3 of this figure is attached to the movable element 4. The sensor 6 may detect when the detectable element 3 is closer to the sensor 6 than a threshold. This may indicate a rotation of the movable element 4. The number of rotations of the movable element 4 (and consequently of the detectable element 3) may thus be used to measure the flow of the outlet fluid.

However, in this example, the movable element 4 is not configured contacted by the outlet fluid as the movable element 4 is arranged outside the main body 10. In this example, the stopcock 100 comprises an actuator 5 placed inside the outlet 13. The actuator 5 is configured to rotate about an actuator rotating axis 151 perpendicular to the outlet length. The actuator 5 also comprises an actuator rotating shaft 51 extending along the actuator rotating axis.

In this figure, the actuator 5 is connected to the movable element 4 that is arranged outside the main body 10. The outlet fluid may move the actuator 5. As the actuator 5 is connected to the movable element 4, the movement of the actuator 5 drives the movable element 4. Accordingly, the movement of actuator 5 drives the detectable element 3.

In this figure, the actuator rotating shaft 51 is connected to the movable element rotating shaft 41 that extends along the movable element rotating axis 141. The movable element rotating axis 141 and actuator rotating axis of this example are parallel. The rotation of the actuator 5 thus causes the rotation of the movable element 4 (and of the detectable element 3) arranged at the outer housing 20. The sensor 6, also arranged at the outer housing 20, may then detect the movement of the detectable element 3.

In this figure, the movement of the actuator rotating shaft 51 is transmitted to the movable element rotating shaft 41. In the example shown, an end 518 of the actuator rotating shaft 51 engages an end 418 of the element rotating shaft 41. In this example, the end 518 of the actuator rotating shaft 51 protrudes from the external surface 132 of the outlet 13. The end 518 may be inserted into a receiver arranged at the end 418 of the element rotating shaft 41. Other types of connection may also be suitable. For example, a gearbox may connect the end 518 of the actuator rotating shaft 51 to the end 418 of the movable element rotating shaft 41.

In the example shown, the actuator 5 is a disc-shaped element, while the movable element 4 is ring-shaped. In other examples, other suitable shapes may also be used.

The outer housing 20 of Figure 4 also comprises an engaging portion 23 to engage the main body 10.

The stopcock of Figure 4 also comprises a flow meter controller 7. The flow meter controller is configured to receive data from the sensor 6, and to determine, based on the data received from the sensor, an amount of outlet fluid supplied by the outlet during a period of time. The flow meter controller 7 is placed in the outer housing 20. The flow meter controller 7 improves the measurement during the period of time. The data received from the sensor comprises a number of rotations of the detectable element 3. In an example, the flow meter controller 7 can be configured to send and/or retrieve data to a non-transitory machine-readable storage medium, or to a central monitoring control system. Additionally, the flow meter assembly 2 can comprise a non-transitory machine-readable storage medium, for example placed in the outer housing 20, to store data from the sensor 6 and/or from the flow meter controller 7, such as the amount of outlet fluid supplied by the outlet during a period of time.

The flow meter assembly 2 further comprises a user interface 29 placed in the outer housing 20. The user interface 29 is connected to the controller 7. The user interface 29 enables the indication of the amount of outlet fluid through the outlet 13. The indication allows the user to know the amount of outlet fluid delivered to the patient, which also helps the monitoring of the fluid delivered to the patient. The user interface 29 may also be configured to activate and deactivate the controller 7. In alternative examples, the user interface may be placed in the main body of the three-way stopcock instead of in the outer housing of the fluid flow meter. In some examples, the user interface 29 is a graphic user interface.

The examples described in Figure 4 can analogously be applied to the medical fluid measuring device according to the second aspect.

Figure 5A illustrates a cross-sectional view of a medical three-way stopcock comprising a flow meter assembly according to an example of the first and/or the second aspect of the present disclosure. The main body 10 of this example may be according to any of the examples disclosed herein. In this example, the amount of outlet fluid is visually indicated by an outlet fluid visual indicator 99. The visual indicator may be mechanically driven. No electrical power may be required to indicate the amount of outlet fluid.

The stopcock 100 of Figure 5A comprises a movable element 4 placed inside the outlet 13. The outlet fluid causes the rotation of the movable element 4. The movable element 4 rotates about a movable element rotating axis 141. The movable element rotating shaft 41 drives a gear assembly 9. The gear assembly 9 is configured to reduce the number of rotations of the movable element 4. The flow meter assembly 2 thus uses a gear assembly 9 for gear reduction. The gear assembly 9 comprises an input gear 91 driveable by the rotation of the movable element 4 and an output gear 93 to drive the outlet fluid visual indicator 99. In this example, the movable element rotating shaft 41 engages the input gear 91. The engagement of the input gear 91 with the shaft 41 eases the driving of the rotation of the shaft 41 to the rotation of the input gear 91. An outer end of the movable element rotating shaft may be connected to one end of the input gear 91. The input gear 91 of this example drives intermediate gears 92, which at the same drive the output gear 93. The output gear 93 drives a shaft 94 that drives the visual indicator 99. In the example of Figure 5A the indicator 99 comprises a set of dials. Each dial can be turned by pegs on the previous dial through a small helper gear (not shown). In other examples, the visual indicator 99 may comprise a needle that may rotate over a calibrated disk.

The outlet output visual indicator 99 may be calibrated to indicate the amount of fluid in millilitres, litres, gallons, or any other unit. The indicator can also be calibrated to indicate the flow of fluid in millilitres per hour, gallons per hour or any other unit.

The flow meter assembly 2 comprises an outer housing 20 removably attached to the main body 10. The gear assembly 9 and the outlet fluid visual indicator 99 are placed in the outer housing 20. The outer housing 20 thus comprises an engaging portion 23 to removably engage the main body. In this example, the main body 10 also comprises an engaging portion. In this example, the engaging portion of the main body 10 comprises a protrusion 232. The engaging portion 23 of the outer housing comprises a recess 932 that receives the protrusion 232. The protrusion 232 may thus be fitted into the recess 932 to establish a snap-fit connection. In other examples, the engaging portion may be according to any of the herein examples.

Figure 5B illustrates a cross-sectional view of a medical three-way stopcock comprising a flow meter assembly according to an example of the first and/or the second aspect of the present disclosure. The example of Figure 5B is similar to the example of Figure 5A; however, in Figure 5B the movable element rotating shaft 41 is not connected to the gear assembly 9.

In this example, the flow meter assembly 2 comprises an elongated member 90 having an inner end 950 and an outer end 960. The elongated member 90 is arranged at an orifice 1390 of the outlet 13. The inner end 950 is configured to be contacted by the movable element 4 during the rotation of the movable element 4. The outer end 960 is configured to engage the input gear 91 to drive the input gear 91. The rotation of the movable element 4 generates a contact with the elongated member 90, which pivots and transmits the movement to the input gear 91. Then, the input gear 91 rotates and the gears of the gear assembly transmit this rotation to the visual indicator 99. Accordingly, the movement of the elongated member 90 drives the gear assembly 9. The elongated member 90 can be a rigid shaft or a flexible cable, and can be made of plastic or a metallic material. The elongated member 90 can additionally comprise a disk element placed in its inner end to improve the contact with the rotation of the movable element 4.

The examples of flow meter assembly 2 described according to the three-way medical stopcock of Figures 5A and 5B, corresponding to the first aspect of the disclosure, can analogously be applied to the medical fluid measuring device according to the second aspect.

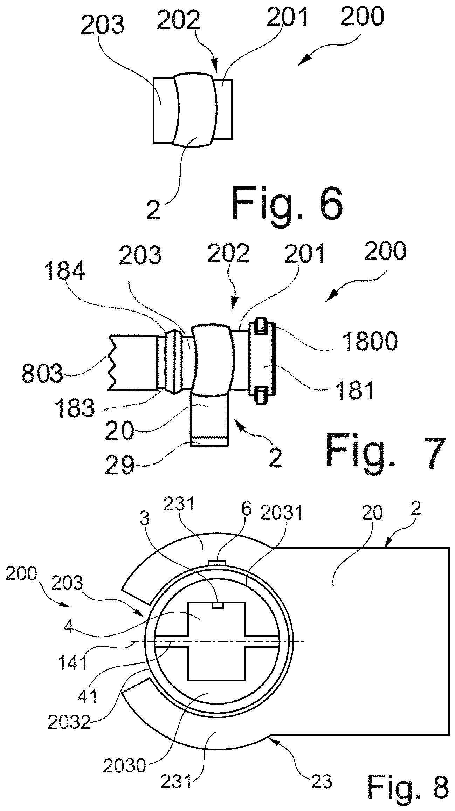

Figure 6 illustrates a medical fluid measuring device 200 for measuring a fluid supplied to a patient according to the second aspect of the disclosure. Analogous elements with the first aspect of the disclosure have been represented with the same reference signs.

The medical fluid measuring device 200 comprises a conduit 202. The conduit 202 allows the distribution and supply of fluid from a fluid source into a patient. The conduit 202 comprises an inlet 201 for receiving the fluid, and an outlet 203 configured to be connected to a proximal end of a catheter 830 to supply the fluid to a patient. The fluid received by the device 200 circulates from the inlet 201 to the outlet 203 to supply the outlet fluid to the patient.

The medical fluid measuring device element 200 acts as a connector element configured to connect a medical tube with a catheter. As the device 200 can be placed as a connector, the flow of outlet fluid can be measured more proximal to the patient than other flow meters that are comprised in the fluid source. Therefore, the accuracy of the amount of fluid supplied to the patient is improved.

Analogously to the outlet of the stopcock of the first aspect, the outlet 203 of the measuring device 200 comprises an external surface 2032 and an internal surface 2031 defining an internal passageway 2030 for conducting the fluid from the inlet 201 to the outlet 203. The conduit 202 comprises an inner diameter of less than 3 mm. The inner diameter corresponds to the internal surface 2031 of the outlet 203.

The medical fluid measuring device 200 comprises a flow meter assembly 2 for measuring the outlet fluid flow, and thus the flow of fluid to be supplied to the patient. The flow meter assembly 2 is configured to measure a fluid flow of less than 100 mL/min. The medical fluid measuring device 200 is thus configured to measure low fluid flows, corresponding to fluid flows commonly employed during medical treatments. The medical fluid measuring device 200 may correspond to any of the examples of flow meter assembly 2 described according with the first aspect of the present disclosure. For example, the flow meter assembly can comprise a detectable element, a sensor to detect the presence of the detectable element, a movable element or any other element previously referenced in accordance with the first aspect.

Figure 7 illustrates a flow meter assembly 2 that comprises an outer housing 20 removably attached to the conduit 202. In this example, the outer housing 20 comprises an engaging portion 23 to removably engage the conduit 202. In particular, the engaging portion 23 removably engages the external surface of the conduit. The engaging portion 23 can be configured to removably engage the outlet 203. In some examples, the engaging portion 23 is configured to partially surround the outlet 203. In some examples, the outer housing 20, comprises a pair of fitting surfaces 231 to press an external surface of the outlet. In other examples, the outer housing 20 and the engaging portions 23 are in accordance with any of the examples of outer housings 20 and engaging portions 23 described in accordance with the first aspect. The conduit 202 can also comprise an engaging portion to removably engage an engaging portion of the outer housing. In the example of Figure 7, the flow meter assembly further comprises a user interface 29 placed in the outer housing 20.

In the example of Figure 7, the inlet 201 comprises an inlet connector 181 for the connection with a medical tube. In the example, the inlet connector 181 comprise protrusions 1800 for further fixing the medical tube into the inlet connector. The expression "medical tube" is to be understood as encompassing a line, a tube, a valve, a stopcock or any other element for supplying, conducting and distributing medical fluid. For example, the inlet connector is configured for the connection with a medical three-way stopcock.

In an example, the medical fluid measuring device 200 comprises a non-return valve (not shown) placed in the inlet 201. The non-return valve can also be placed on the outlet 203. The non-return valve ensures that the fluid flows through the device 200 from the inlet 201 to the outlet 203 by stopping the pass of fluid through the inlet 201, thus impeding the flow of fluid in a reverse direction from the outlet 203 to the inlet 201.This flow in a reverse direction may occur, for example, when the inlet is not connected to any medical tube while fluid remains in the conduit or in the catheter connected to the outlet. In that case, the pressure on the inlet corresponds to the atmospheric pressure, which is lower than the pressure of the fluid in the outlet. Without the non-return valve, fluid in the conduit 202 or in the catheter connected to the outlet 203 could flow in the reverse direction exiting the conduit 202 by its inlet 201.

The outlet 203 also comprises an outlet connector 183 configured to engage a catheter 830. In this example, the outlet connector 183 comprises a protrusion part 184 to cause a dimensional interference with the proximal end of a catheter 830. The external surface of the outlet has an external diameter such that the inner surface of the catheter is fixed outside the external surface of the outlet 203. The outlet connector 183 may be in accordance with to the outlet connectors 183 described in accordance with the first aspect.

Figure 8 illustrates an example of a medical fluid measuring device 200 in which the flow meter assembly 2 comprises a detectable element 3 placed in the movable element 4 and a sensor 6 to detect the presence of the detectable element 3. The movement of the movable element 4 moves the detectable element 3. The sensor 6 detects the detectable element 3 when the movement of the movable element 4 leads the detectable element 3 to a position close enough to the sensor 6 to be detected. This detection allows the flow meter assembly 2 to measure the flow of the outlet fluid. The amount of outlet fluid passing through the movable element may thus be detected. In Figure 8, the detectable element 3 and the movable element 4 are placed inside the outlet 203, configured to rotate about a movable element rotating axis 141. The movable element 4 comprises a movable element rotating shaft 41 extending along the movable element rotating axis 141.

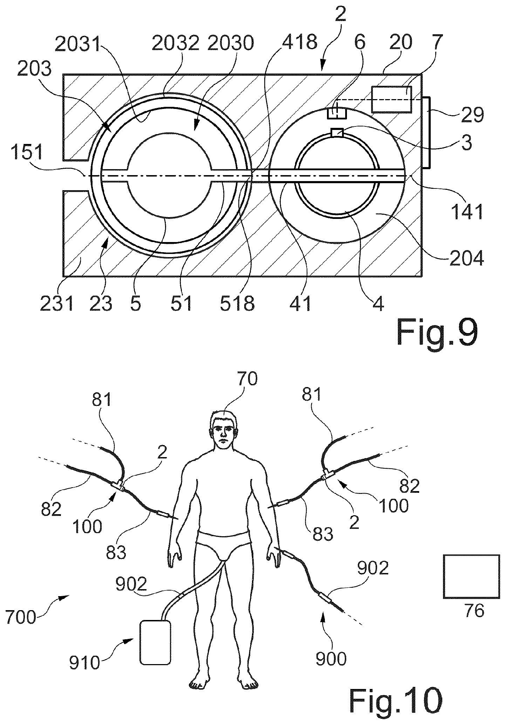

Figure 8 also illustrate that the outer housing 20 comprises an engaging portion 23 to removably engage the conduit 202. The engaging portion 23 is configured to removably engage the outlet 203 and is configured to at least partially surround the outlet 203. The engaging portion 23 of Figure 9 comprises a pair of fitting surfaces 231 to press an engaging portion of the conduit 202. The outlet 203 may thus be clamped by the pair of fitting surfaces 231. Alternatively, the engaging portion 23 may be in accordance with any of the examples and alternatives disclosed in accordance with the first aspect.

Figure 9 illustrates an example of a flow meter assembly 2 in which the detectable element 3 is mounted on the movable element 4 and arranged outside the outlet 203. The flow meter assembly 2 comprises an outer housing 20 removably attached to the conduit 202. The outer housing 20 houses the movable element 4, the detectable element 3 and the sensor 6. In this example, the device 200 comprises an actuator 5 placed inside the outlet 203.

As previously disclosed, the medical fluid measuring device 200 may comprise any of the examples of flow meter assembly 2 described in connection with the first aspect of the present disclosure. For example, the flow measuring system may comprise no detectable element or sensor and comprise a movable element placed inside the outlet. In this example, the movable element can be configured to be contacted by the outlet fluid for rotating the movable element about a movable element rotating axis. In another example, the flow meter assembly may comprise an outlet fluid visual indicator and a gear assembly. The gear assembly may also comprise an input gear driveable by the rotation of the movable element; an output gear to drive the outlet fluid visual indicator; the gear assembly configured to reduce the number of rotations from the input gear to the output gear.

In the example of Figure 9, the medical fluid measuring device 200 also comprises a flow meter controller 7. The flow meter controller 7 is in accordance with the flow meter controller of the first aspect.

Figure 10 illustrates a fluid flow monitoring system 700 for monitoring a patient body fluid balance of a third aspect of the disclosure.

The fluid flow monitoring system 700 comprises one or more medical fluid measuring devices 200 according to second aspect, and/or one or more medical three-way stopcocks 100 according to the first aspect.

In the example of Figure 10, the fluid flow monitoring system 700 comprises two medical three-way stopcocks 100. Each stopcock 100 comprises a flow meter assembly 2 to measure the outlet fluid flow of each stopcock 100. The flow meter assembly 2 of each stopcock 2 allows the measurement of the amount of outlet fluid supplied to the patient by that stopcock in a period of time. This measurement of each of the stopcocks 100 can be used to determine the patient body fluid balance. The patient body fluid balance corresponds to the difference between the amount of outlet fluid supplied by each stopcock 100, and the output fluid removed from the patient. In another examples, the fluid flow monitoring system 700 may comprise a different number of stopcocks 100. Although Figure 10 illustrates an example of fluid flow monitoring system 700 in which the outlet fluid which is inputted to the patient is supplied through one or more stopcocks 100, it is to be understood that the outlet fluid can also be supplied through one or more medical fluid measuring devices 200.

In the example shown, the first and second inlets 11, 12 of each stopcock 100 are connected to a first inlet line 81 and to a second inlet line 82. Fluid sources may be connected to the inlet lines. The outlet 13 of each stopcock 100 is connected to an outlet line 83 that is connected to an intravenous catheter to supply outlet fluid to the patient 70.

The fluid flow monitoring system also comprises one or more body patient output fluid meters 902 for measuring the flow of fluid outputted from the patient body. Each body patient output fluid meter 902 is configured to measure a flow of fluid emanated, and thus, removed from the patient. In this example, the fluid flow monitoring system comprises an output stopcock 900 and a urine collection bag 910. The body patient output fluid meters 902 are connected to the urine collection bag 910 and to the output stopcock 900. The body patient output fluid meters 902 may measure the output body patient fluid that circulates through each of the patient outputs.

In some examples, the output fluid meters 902 may be according to any of the examples of flow meter assemblies 2 of the medical three-way stopcocks 100 or medical fluid measuring devices 200 herein disclosed. In other examples, other suitable flow fluid meters may be used.

The fluid flow monitoring system 700 comprises a monitoring system controller 76. The monitoring system controller 76 is configured to obtain an amount of outlet fluid supplied by the outlet of the one or more stopcocks 100 during a period of time. Based on the obtained amount of outlet fluid supplied by the outlet of the one or more stopcocks, the controller 76 can determine an amount of fluid inputted into the patient body during the period of time. The monitoring system controller 76 is also configured to obtain, from the one or more body patient output fluid meters 902, an amount of fluid outputted from the patient body during the period of time.

The monitoring system controller 76 is communicatively coupled to the flow meter assembly 2 of each of the stopcocks 100 and to the body patient fluid meters 902. A wireless connection may be established between the meters 2, 902 and the controller 76. In other examples, the flow meter assemblies 2 and the body patient fluid meters 902 are connected to the controller 76 through a wired connection. The controller 76 may also be coupled to flow meter controllers 7 corresponding to the stopcocks 100, 900.

The monitoring system controller 76 is also configured to compare the amount of fluid inputted into the patient's body during the period of time with the amount of fluid outputted from a patient body during that period of time and to determine the patient body fluid balance during that period of time. The input of fluid into the patient increases the body fluid balance, while the output of fluid from the patient decreases the body fluid balance. This comparison allows the medical staff user to have control of the balance, thus allowing the user to evaluate whether the patient's body fluid balance is adequate and if any modifications in the amount of fluid supplied or removed from the patient have to be made in order to obtain a desired patient body fluid balance.

In some examples, the monitoring system controller 76 is configured to receive, from the flow meter assemblies 2, e.g. from the flow meter controllers 7, and the body patient fluid meters 902, a plurality of data at different sampling times during a predetermined period of time. The expression "a period of time" is to be understood as any period of time during which the inputs of fluid into the patient, the output of fluid from the patient, and/or the patient body fluid patient is selected to be controlled or monitored.

The monitoring system controller 76 may sample the outlet fluid during a predetermined period of time. Fluid outputted from the patient's body may also be sampled during the predetermined period of time. The monitoring system controller 76 may then determine a patient body fluid balance during sampling times. The monitoring system controller may then determine an accumulated fluid body balance based on the sum of the patient body fluid balance according to the different sampling times.

In some examples, the monitoring system controller 76 is further configured to send data of the fluid flow monitoring system to a non-transitory machine-readable storage medium. In addition, the monitoring system controller is further configured to retrieve data of the fluid flow monitoring system from the non-transitory machine-readable storage medium. The storage medium may allow the medical staff user to store data and retrieve it at any time, allowing a control the evolution of the data stored within, and to generate flowcharts and graphics to evaluate the evolution of different parameters over time.

The data of the fluid flow monitoring system include one or more different parameters. The data of the fluid flow monitoring system comprises at least one of:
- An amount of outlet fluid supplied by the outlet of the one or more stopcocks during the period of time;
- An amount of fluid inputted into the patient body during the period of time;
- An amount of fluid outputted from the patient body during the period of time;
- A patient body fluid balance during the period of time.

The monitoring system controller 76 can be connected to the different flow meters 2, 902, so that data from the different flow meters 2, 902 can be stored from the flow meters 2, 902 to the non-transitory machine-readable storage medium. In addition, the different flow meters 2 may be directly connected to the non-transitory machine-readable storage medium.

In some examples, the monitoring system controller 76 may be connected to a monitoring system user interface. The monitoring system user interface can be connected to the non-transitory machine-readable storage medium as well. The user interface can be configured to allow the medical personnel to introduce data such as data related to non-intravenous fluid inputs and/or outputs of the patient (for example, oral fluid intake such as food, beverages, or fluid loss due to vomiting, bleeding or excessive sweating) during the period of time. The user interface may be a CPU, an mobile application program, a tablet, or any similar element.

In some examples, the monitoring system controller 76 is further configured to compare the patient body fluid balance with a predetermined patient body fluid balance upper threshold value and with a predetermined patient body fluid balance lower threshold value. This comparison allows an easier evaluation of whether the patient body fluid balance at a certain moment may imply a risk for patient. Therefore, this comparison allows the medical staff user to modify the flow of fluid delivered into the stopcocks, and therefore supplied to the patient, in order to reduce the risk of having complications to the patient. If the patient body fluid balance is lower than the predetermined patient body fluid balance lower threshold value, the patient is reducing the fluid inside him or her. This may indicate a risk of dehydration. The medical staff user may thus decide to input more fluid into the patient. If the patient body fluid balance is higher than the predetermined patient body fluid balance upper threshold value, the patient is increasing the fluid inside him or her. This may indicate a risk of sepsis. The medical staff user may thus decide to input less fluid into the patient or to provide the patient with diuretic medication. The intravenous treatment of the patient can therefore be adapted to obtain a desired patient body fluid balance.

These threshold values may be stored in a database. The monitoring system controller 76 may thus obtain the threshold values from the database. The threshold values may be determined by experimental data. In some examples, the threshold values may also be obtained from a user interface device. In some examples, the database may be comprised in the non-transitory machine-readable storage medium.

In some examples, the monitoring system controller 76 can be configured to further generate a signal when the patient body fluid balance is lower to the predetermined patient body fluid balance lower threshold value and/or greater than the predetermined patient body fluid balance upper threshold value. This signal can be a visual signal, such as light emitted from a light-emitting diode (LED), an acoustic signal, or any other signal.

In some examples, the controller 76 may be configured to output the result of the comparison between the patient body fluid balance and the threshold values. For example, the result of this comparison may be outputted in a user interface device connected to the controller 76.

For reasons of completeness, various aspects of the present disclosure are set out in the following numbered clauses:
Clause 1. A medical three-way stopcock comprising:
   a main body comprising:
      a first inlet for receiving a first fluid,
      a second inlet for receiving a second fluid,
      an outlet,
      a central part fluidically connecting the first inlet and the second inlet to the outlet for blending the first fluid and the second fluid to obtain an outlet fluid,
      wherein the outlet is configured to supply the outlet fluid to a patient; and
   a flow meter assembly for measuring a flow of outlet fluid.
Clause 2. A medical three-way stopcock according to clause 1, wherein the outlet comprises an inner diameter of less than 3 mm.
Clause 3. A medical three-way stopcock according to any of clauses 1 to 2, wherein the first inlet and/or the second inlet comprise an inner diameter of less than 3 mm.
Clause 4. A medical three-way stopcock according to any of clauses 1 to 3, wherein the flow meter assembly is configured to measure a fluid flow of less than 100 mL/min.
Clause 5. A medical three-way stopcock according to any of clauses 1 to 4, wherein the three-way stopcock comprises a valve to enable and disable the pass of the first fluid and the second fluid into the central part of the main body.
Clause 6. A medical three-way stopcock according to any of clauses 1 to 5, wherein the three-way stopcock comprises a non-return valve placed in the first inlet and/or the second inlet and/or the outlet.
Clause 7. A medical three-way stopcock according to any of clauses 1 to 6, wherein the flow meter assembly comprises a detectable element and a sensor to detect the presence of the detectable element.
Clause 8. A medical three-way stopcock according to clause 7, wherein the detectable element is an electromagnet, and the sensor is an electromagnetic sensor.
Clause 9. A medical three-way stopcock according to any of clauses 7 or 8, wherein the three-way stopcock comprises a movable element, and wherein the detectable element is placed in the movable element.
Clause 10. A medical three-way stopcock according to clause 9, wherein the movable element is configured to rotate about a movable element rotating axis.
Clause 11. A medical three-way stopcock according to clause 10, wherein the outlet extends an outlet length from an outlet proximal end to an outlet distal end; and wherein the movable element rotating axis is perpendicular to the outlet length.
Clause 12. A medical three-way stopcock according to any of clauses 9 to 11, wherein the movable element is a disc, a rotor, an helix or a turbine.
Clause 13. A medical three-way stopcock according to any of clauses 7 to 12, wherein the detectable element is placed inside the outlet.
Clause 14. A medical three-way stopcock according to any of clauses 9 to 13, wherein the detectable element is placed in the movable element, wherein the movable element is placed inside the outlet, and wherein the movable element is configured to be contacted by the outlet fluid for moving the movable element.
Clause 15. A medical three-way stopcock according to any of clauses 7 to 12, wherein the detectable element is placed outside the main body.
Clause 16. A medical three-way stopcock according to clause 15, wherein the three-way stopcock comprises an actuator placed inside the outlet, and wherein the actuator is configured to be contacted by the outlet fluid for moving the actuator, and wherein the movement of the actuator drives the movement of the detectable element.
Clause 17. A medical three-way stopcock according to clause 16, further comprising:
   a movable element arranged outside the main body, wherein the detectable element is arranged on the movable element; and
   wherein the actuator is connected to the movable element.
Clause 18. A medical three-way stopcock according to clause 17, wherein the actuator comprises an actuator rotating shaft extending along an actuator rotating axis; and wherein the actuator is configured to rotate about the actuator rotating axis.
Clause 19. A medical three-way stopcock according to clause 18, wherein the movable element comprises a movable element rotating shaft extending along a movable element rotating axis; wherein the movable element is configured to rotate about the movable element rotating axis.
Clause 20. A medical three-way stopcock according to clause 19, wherein the movable element rotating shaft is connected to the actuator rotating shaft.
Clause 21. A medical three-way stopcock according to any of clauses 16 to 20, wherein the actuator is a disc, a rotor, an helix or a turbine.
Clause 22. A medical three-way stopcock according to any of clauses 7 to 21, wherein the stopcock comprises a flow meter controller configured to:
   receive data from the sensor,
   determine, based on the data received from the sensor, an amount of outlet fluid supplied by the outlet during a period of time.
Clause 23. A medical three-way stopcock according to clause 22 when depended on clause 10, wherein to receive data from the sensor comprises to receive a number of rotations of the detectable element placed in the movable element.
Clause 24. A medical three-way stopcock according to any of clauses 7 to 23, wherein the flow meter assembly comprises an outer housing removably attached to the main body.
Clause 25. A medical three-way stopcock according to clause 24, wherein the detectable element is placed in the outer housing.
Clause 26. A medical three-way stopcock according to any of clauses 24 to 25, wherein the sensor is placed in the outer housing.
Clause 27. A medical three-way stopcock according to any of clauses 24 to 26, wherein the controller according to any of clauses 22 to 23 is placed in the outer housing.
Clause 28. A medical three-way stopcock according to any of clauses 24 to 27, wherein the flow meter assembly further comprises a user interface placed in the outer housing.
Clause 29. A medical three-way stopcock according to any of clauses 24 to 28, wherein the outer housing comprises an engaging portion to removably engage the main body.
Clause 30. A medical three-way stopcock according to clause 29, wherein the engaging portion is configured to removably engage the outlet.
Clause 31. A medical three-way stopcock according to clause 30, wherein the engaging portion is configured to at least partially surround the outlet.
Clause 32. A medical three-way stopcock according to clause 31, wherein the engaging portion comprises a pair of fitting surfaces to press an external surface of the outlet.
Clause 33. A medical three-way stopcock according to any of clauses 29 to 32, wherein the main body comprises an engaging portion to removably engage the engaging portion of the outer housing.
Clause 34. A medical three-way stopcock according to clause 33, wherein the engaging portion of the outer housing and the engaging portion of the main body forms a snap-fit connection.
Clause 35. A medical three-way stopcock according to any of clauses 20 to 34, wherein the main body is a fungible component and wherein the outer housing is configured to be removably attached to another main body to reuse the outer housing.
Clause 36. A medical three-way stopcock according to any of clauses 1 to 6, wherein the stopcock comprises:
   a movable element placed inside the outlet, the movable element configured to be contacted by the outlet fluid for rotating the movable element about a movable element rotating axis, and wherein the flow meter assembly comprises:
   - an outlet fluid visual indicator,
   - a gear assembly comprising:
      ∘ an input gear driveable by the rotation of the movable element;
      ∘ an output gear to drive the outlet fluid visual indicator;
      ∘ wherein the gear assembly is configured to reduce the number of rotations from the input gear to the output gear.
Clause 37. A medical three-way stopcock according to clause 36, wherein the movable element comprises a movable element rotating shaft extending along the movable element rotating axis.
Clause 38. A medical three-way stopcock according to clause 37, wherein the outlet extends an outlet length from an outlet proximal end to an outlet distal end; and wherein the movable element rotating axis is perpendicular to the outlet length.
Clause 39. A medical three-way stopcock according to any of clauses 36 to 37, wherein the input gear is configured to engage the movable element rotating shaft.
Clause 40. A medical three-way stopcock according to any of clauses 37 to 38, wherein flow meter assembly comprises an elongated member comprising:
   - an inner end configured to be contacted by the movable element during the rotation of the movable element; and an outer end configured to engage the input gear to drive the input gear.
Clause 41. A medical three-way stopcock according to any of clauses 36 to 39, wherein the gear assembly comprises one or more intermediate gears between the input gear and the output gear.
Clause 42. A medical three-way stopcock according to any of clauses 36 to 41, wherein the stopcock comprises an outer housing, the outlet fluid visual indicator and the gear assembly being placed at the outer housing.
Clause 43. A medical three-way stopcock according to clause 42, wherein the flow meter assembly further comprises a user interface placed in the outer housing.
Clause 44. A medical three-way stopcock according to any of clauses 42 to 43, wherein the outer housing comprises an engaging portion to removably engage the main body.
Clause 45. A medical three-way stopcock according to clause 44, wherein the engaging portion is configured to removably engage the outlet.
Clause 46. A medical three-way stopcock according to any of clauses 44 to 45, wherein the engaging portion is configured to at least partially surround the outlet.
Clause 47. A medical three-way stopcock according to any of clauses 44 to 46, wherein the engaging portion comprises a pair of fitting surfaces to press an external surface of the outlet.
Clause 48. A medical three-way stopcock according to any of clauses 44 to 47, wherein the main body comprises an engaging portion to removably engage the engaging portion of the outer housing.
Clause 49. A medical three-way stopcock according to clause 48, wherein the engaging portion of the outer housing and the engaging portion of the main body forms a snap-fit connection.
Clause 50. A medical three-way stopcock according to any of clauses 42 to 49, wherein the main body is a fungible component and wherein the outer housing is configured to be removably attached to another main body to reuse the outer housing.
Clause 51. A medical fluid measuring device for measuring a fluid supplied to a patient comprising:
   a conduit comprising an inner diameter of less than 3 mm, wherein the conduit comprises:
      an inlet for receiving the fluid, and
      an outlet configured to be connected to a proximal end of a catheter to supply an outlet fluid to a patient; and
   a flow meter assembly for measuring the outlet fluid flow, wherein the flow meter assembly is configured to measure a fluid flow of less than 100 mL/min.
Clause 52. A medical fluid measuring device according to clause 51, wherein the inlet comprises an inlet connector for the connection with a medical tube.
Clause 53. A medical fluid measuring device according to clause 52, wherein the inlet connector is configured for the connection with a medical valve or a medical three-way stopcock.
Clause 54. A medical fluid measuring device according to any of clauses 51 to 53, wherein the outlet comprises an outlet connector for the connection with a catheter.
Clause 55. A medical fluid measuring device according to any of clauses 51 to 54, wherein the flow meter assembly comprises a detectable element and a sensor to detect the presence of the detectable element.
Clause 56. A medical fluid measuring device according to clause 55, wherein the detectable element is an electromagnet, and the sensor is an electromagnetic sensor.
Clause 57. A medical fluid measuring device according to any of clauses 55 or 56, wherein the medical fluid measuring device comprises a movable element, and wherein the detectable element is placed in the movable element.
Clause 58. A medical fluid measuring device according to clause 57, wherein the movable element is configured to rotate about a movable element rotating axis.
Clause 59. A medical fluid measuring device according to clause 58, wherein the outlet extends an outlet length from an outlet proximal end to an outlet distal end; and wherein the movable element rotating axis is perpendicular to the outlet length.
Clause 60. A medical fluid measuring device according to any of clauses 57 to 59, wherein the movable element is a disc, a rotor, an helix or a turbine.
Clause 61. A medical fluid measuring device according to any of clauses 55 to 60, wherein the detectable element is placed inside the outlet.
Clause 62. A medical fluid measuring device according to any of clauses 57 to 61, wherein the detectable element is placed in the movable element, wherein the movable element is placed inside the outlet, and wherein the movable element is configured to be contacted by the outlet fluid.
Clause 63. A medical fluid measuring device according to any of clauses 55 to 60, wherein the detectable element is placed outside the conduit.
Clause 64. A medical fluid measuring device according to clause 63, wherein the medical fluid measuring device comprises an actuator placed inside the outlet, and wherein the actuator is configured to be contacted by the outlet fluid for moving the actuator, and wherein the movement of the actuator drives the movement of the detectable element.
Clause 65. A medical fluid measuring device according to clause 64, further comprising:
   a movable element arranged outside the main body, wherein the detectable element is arranged on the movable element; and
   wherein the actuator is connected to the movable element.
Clause 66. A medical fluid measuring device according to clause 65, wherein the actuator comprises an actuator rotating shaft extending along an actuator rotating axis; and wherein the actuator is configured to rotate about the actuator rotating axis.
Clause 67. A medical fluid measuring device according to clause 66, wherein the movable element comprises a movable element rotating shaft extending along a movable element rotating axis; wherein the movable element is configured to rotate about the movable element rotating axis.
Clause 68. A medical fluid measuring device according to clause 67, wherein the movable element rotating shaft is connected to the actuator rotating shaft.
Clause 69. A medical fluid measuring device according to any of clauses 64 to 68, wherein the actuator is a disc, a rotor, an helix or a turbine.
Clause 70. A medical fluid measuring device according to any of clauses 55 to 69, wherein the medical fluid measuring device comprises a flow meter controller configured to:
   receive data from the sensor,
   determine, based on the data received from the sensor, an amount of fluid supplied to the patient during a period of time.
Clause 71. A medical fluid measuring device according to clause 70 when depended on clause 58, wherein to receive data from the sensor comprises to receive a number of rotations of the detectable element placed in the movable element.
Clause 72. A medical fluid measuring device according to any of clauses 55 to 71, wherein the flow meter assembly comprises an outer housing removably attached to the conduit.
Clause 73. A medical fluid measuring device according to clause 72, wherein the detectable element is placed in the outer housing.
Clause 74. A medical fluid measuring device according to any of clauses 72 to 73, wherein the sensor is placed in the outer housing.
Clause 75. A medical fluid measuring device according to any of clauses 72 to 74, wherein the controller according to any of clauses 70 to 71 is placed in the outer housing.
Clause 76. A medical fluid measuring device according to any of clauses 72 to 75, wherein the flow meter assembly further comprises a user interface placed in the outer housing.
Clause 77. A medical fluid measuring device according to any of clauses 72 to 76, wherein the outer housing comprises an engaging portion to removably engage the conduit.
Clause 78. A medical fluid measuring device according to clause 77, wherein the engaging portion is configured to removably engage the outlet.
Clause 79. A medical fluid measuring device according to clause 78, wherein the engaging portion is configured to at least partially surround the outlet.
Clause 80. A medical fluid measuring device according to clause 79, wherein the engaging portion comprises a pair of fitting surfaces to press an external surface of the outlet.
Clause 81. A medical fluid measuring device according to any of clauses 77 to 80, wherein the conduit comprises an engaging portion to removably engage the engaging portion of the outer housing.
Clause 82. A medical fluid measuring device according to clause 81, wherein the engaging portion of the outer housing and the engaging portion of the conduit forms a snap-fit connection.
Clause 83. A medical fluid measuring device according to any of clauses 72 to 82, wherein the conduit is a fungible component and wherein the outer housing is configured to be removably attached to another conduit to reuse the outer housing.
Clause 84. A medical fluid measuring device according to any of clauses 51 to 54, wherein the medical fluid measuring device comprises:
   a movable element placed inside the outlet, the movable element configured to be contacted by the outlet fluid for rotating the movable element about a movable element rotating axis, and wherein the flow meter assembly comprises:
   - an outlet fluid visual indicator,
   - a gear assembly comprising:
      ∘ an input gear driveable by the rotation of the movable element;
      ∘ an output gear to drive the outlet fluid visual indicator;
      ∘ wherein the gear assembly is configured to reduce the number of rotations from the input gear to the output gear.
Clause 85. A medical fluid measuring device according to clause 84, wherein the movable element comprises a movable element rotating shaft extending along the movable element rotating axis.
Clause 86. A medical fluid measuring device according to clause 85, wherein the outlet extends an outlet length from an outlet proximal end to an outlet distal end; and wherein the movable element rotating axis is perpendicular to the outlet length.
Clause 87. A medical fluid measuring device according to any of clauses 85 to 86, wherein the input gear is configured to engage the movable element rotating shaft.
Clause 88. A medical fluid measuring device according to any of clauses 85 to 86, wherein flow meter assembly comprises an elongated member comprising:
   - an inner end configured to be contacted by the movable element during the rotation of the movable element; and an outer end configured to engage the input gear to drive the input gear.
Clause 89. A medical fluid measuring device according to any of clauses 84 to 88, wherein the gear assembly comprises one or more intermediate gears between the input gear and the output gear.
Clause 90. A medical fluid measuring device according to any of clauses 84 to 89, wherein the medical fluid measuring device comprises an outer housing, the outlet fluid visual indicator and the gear assembly being placed at the outer housing.
Clause 91. A medical fluid measuring device according to any of clauses 84 to 90, wherein the medical fluid measuring device comprises an outer housing, the outlet fluid visual indicator and the gear assembly being placed at the outer housing.
Clause 92. A medical fluid measuring device according to clause 91, wherein the flow meter assembly further comprises a user interface placed in the outer housing.
Clause 93. A medical fluid measuring device according to any of clauses 91 to 92, wherein the outer housing comprises an engaging portion to removably engage the conduit.
Clause 94. A medical fluid measuring device according to clause 93, wherein the engaging portion is configured to removably engage the outlet.
Clause 95. A medical fluid measuring device according to any of clauses 93 to 94, wherein the engaging portion is configured to at least partially surround the outlet.
Clause 96. A medical fluid measuring device according to any of clauses 93 to 95, wherein the engaging portion comprises a pair of fitting surfaces to press an external surface of the outlet.
Clause 97. A medical fluid measuring device according to any of clauses 93 to 96, wherein the main body comprises an engaging portion to removably engage the engaging portion of the outer housing.
Clause 98. A medical fluid measuring device according to clause 97, wherein the engaging portion of the outer housing and the engaging portion of the main body forms a snap-fit connection.
Clause 99. A medical fluid measuring device according to any of clauses 91 to 98, wherein the conduit is a fungible component and wherein the outer housing is configured to be removably attached to another conduit to reuse the outer housing.
Clause 100. A medical fluid measuring device according to any of clauses 51 to 99, wherein the device comprises a valve to enable and disable the pass of fluid through the conduit.
Clause 101. A medical fluid measuring device according to any of clauses 51 to 100, wherein the device comprises a non-return valve placed in the inlet and/or the outlet.
Clause 102. Fluid flow monitoring system for monitoring a patient body fluid balance, the fluid flow monitoring system comprising:
   one or more medical fluid measuring devices according to any of clauses 51 to 101 or 108, and/or one or more medical three-way stopcocks according to any of clauses 1 to 50 or 109;
   one or more body patient output fluid meters for measuring the flow of fluid outputted from the patient body;
   a monitoring system controller configured to:
      obtain an amount of outlet fluid supplied by the outlet of the one or more medical fluid measuring devices and/or by the one or more medical three-way stopcocks during a period of time;
         determine, based on the obtained amount of outlet fluid, an amount of fluid inputted into the patient body during the period of time;
      obtain, from the one or more body patient output fluid meters, an amount of fluid outputted from the patient body during the period of time;
      compare the amount of fluid inputted into the patient body during the period of time with the amount of fluid outputted from a patient body during the period of time; and
      determine, based on the comparison, the patient body fluid balance during the period of time.
Clause 103. Fluid flow monitoring system according to clause 102, wherein the monitoring system controller is further configured to:
   compare the patient body fluid balance with a predetermined patient body fluid balance upper threshold value and with a predetermined patient body fluid balance lower threshold value.
Clause 104. Fluid flow monitoring system according to clause 103, wherein the monitoring system controller is further configured to:
   - generate a signal when the patient body fluid balance is lower to the predetermined patient body fluid balanced lower threshold value and/or greater than the predetermined patient body fluid balance upper threshold value.
Clause 105. Fluid flow monitoring system according to any of clauses 102 to 104, wherein the monitoring system controller is further configured to send data of the fluid flow monitoring system to a non-transitory machine-readable storage medium.
Clause 106. Fluid flow monitoring system according to any of clauses 102 to 105, wherein the monitoring system controller is further configured to retrieve data of the fluid flow monitoring system from a non-transitory machine-readable storage medium.
Clause 107. Fluid flow monitoring system according to any of clauses 102 to 106, wherein the data of the fluid flow monitoring system comprises at least one of:
   an amount of output fluid supplied by the outlet of the one or more medical fluid measuring devices or by the one or more stopcocks during the period of time;
   an amount of fluid inputted into the patient body during the period of time;
   an amount of fluid outputted from the patient body during the period of time;
   a patient body fluid balance during the period of time.
Clause 108. A medical three-way stopcock according to any of clauses 1 to 50, wherein the outlet is configured to receive fluid emanated from the patient.
Clause 109. A medical fluid measuring device according to any of clauses 51 to 108, wherein the medical fluid measuring device is configured for measuring a fluid emanated from the patient, the outlet being configured to be connected to a proximal end of a catheter to receive an outlet fluid from the patient, and wherein the inlet is configured for receiving the outlet fluid.

Although only a number of examples have been disclosed herein, other alternatives, modifications, uses and/or equivalents thereof are possible. Furthermore, all possible combinations of the described examples are also covered. Thus, the scope of the present disclosure should not be limited by particular examples, but should be determined only by a fair reading of the claims that follow.

## Claims

1. A medical fluid measuring device (200) for measuring a fluid supplied to a patient comprising:
a conduit (202) comprising an inner diameter of less than 3 mm, wherein the conduit (202) comprises:
an inlet (201) for receiving the fluid, and
an outlet (203) configured to be connected to a proximal end of a catheter to supply an outlet fluid to a patient; and
a flow meter assembly (2) for measuring the outlet fluid flow, wherein the flow meter assembly (2) is configured to measure a fluid flow of less than 100 mL/min.

2. A medical fluid measuring device (200) according to claim 1, wherein the inlet (201) comprises an inlet connector (181) for the connection with a medical tube.

3. A medical fluid measuring device (200) according to claim 1, wherein the outlet (203) comprises an outlet connector (183) for the connection with a catheter.

4. A medical fluid measuring device (200) according to any of claims 1 to 3, wherein the flow meter assembly (2) comprises a detectable element (3) and a sensor (6) to detect the presence of the detectable element (3).

5. A medical fluid measuring device (200) according to claim 4, wherein the medical fluid measuring device (200) comprises a movable element (4), and wherein the detectable element (3) is placed in the movable element (4).

6. A medical fluid measuring device (200) according to claim 5, wherein the movable element (4) is configured to rotate about a movable element rotating axis (141).

7. A medical fluid measuring device (200) according to any of claims 3 to 6, wherein the detectable element (3) is placed in the movable element (4), wherein the movable element (4) is placed inside the outlet (203), and wherein the movable element (4) is configured to be contacted by the outlet fluid.

8. A medical fluid measuring device (200) according to any of claims 3 to 6, wherein the detectable element (3) is placed outside the conduit (202), wherein the medical fluid measuring device (200) comprises an actuator (5) placed inside the outlet (203), wherein the actuator (5) is configured to be contacted by the outlet fluid for moving the actuator (5), and wherein the movement of the actuator (5) drives the movement of the detectable element (3).

9. A medical fluid measuring device according to any of claims 4 to 8, wherein the medical fluid measuring device (200) comprises a flow meter controller (7) configured to:
receive data from the sensor (6),
determine, based on the data received from the sensor (6), an amount of fluid supplied to the patient during a period of time.

10. A medical fluid measuring device (200) according to any of claims 4 to 9, wherein the flow meter assembly (2) comprises an outer housing (20) removably attached to the conduit (202).

11. A medical fluid measuring device (200) according to claim 10, wherein the detectable element 3 and/or the sensor 6 is placed in the outer housing (20).

12. A medical fluid measuring device (200) according to any of claims 10 to 11, wherein the conduit (202) is a fungible component and wherein the outer housing (20) is configured to be removably attached to another conduit (202) to reuse the outer housing.

13. A medical fluid measuring device (200) according to any of claims 1 to 12, wherein the device (200) comprises a non-return valve placed in the inlet (201).

14. Fluid flow monitoring system (700) for monitoring a patient body fluid balance, the fluid flow monitoring system comprising:
one or more medical fluid measuring devices (200) according to any of claims 1 to 13;
one or more body patient output fluid meters (902) for measuring the flow of fluid outputted from the patient body;
a monitoring system controller (76) configured to:
obtain an amount of outlet fluid supplied by the outlet (203) of the one or more medical fluid measuring devices (200) during a period of time;
determine, based on the obtained amount of outlet fluid supplied by the outlets (203) of the one or more medical fluid measuring devices (200), an amount of fluid inputted into the patient body during the period of time;
obtain, from the one or more body patient output fluid meters (902), an amount of fluid outputted from the patient body during the period of time;
compare the amount of fluid inputted into the patient body during the period of time with the amount of fluid outputted from a patient body during the period of time; and
determine, based on the comparison, the patient body fluid balance during the period of time.

15. Fluid flow monitoring system (700) according to claim 14, wherein the monitoring system controller is further configured to:
compare the patient body fluid balance with a predetermined patient body fluid balance upper threshold value and with a predetermined patient body fluid balance lower threshold value.
